# EUROPEAN PATENT SPECIFICATION

(11) **EP 2 355 723 B1**
(45) Date of publication and mention of the grant of the patent: **26.12.2018**
(21) Application number: 09760046.4
(22) Date of filing: 10.11.2009
(51) Int. Cl.: A61B 17/70

(54) **GROWTH DIRECTED VERTEBRAL FIXATION SYSTEM WITH DISTRACTIBLE CONNECTOR(S) AND APICAL CONTROL**
AUF WACHSTUM GERICHTETES WIRBELFIXATIONSSYSTEM MIT ABLENKBAREM VERBINDUNGSGLIED (ABLENKBAREN VERBINDUNGSGLIEDERN) UND APIKALER KONTROLLE
SYSTÈME DE FIXATION VERTÉBRAL À DES FINS DE CROISSANCE AVEC CONNECTEUR(S) RÉGLABLE(S) ET CONTRÔLE APICAL

(30) Priority: 11.11.2008 EG 2008111840
(43) Date of publication of application: 17.08.2011
(73) Proprietor: K2M, Inc., Leesburg, VA 20175 (US)
(72) Inventor: ELSEBAIE, Hazem B., Elmohandessine Giza 12411 (EG); AKBARNIA, Behrooz, La Jolla California 92038 (US)
(74) Representative: Vossius & Partner Patentanwälte Rechtsanwälte mbB
(86) International application number: PCT/US2009/063833
(87) International publication number: WO 2010/056650

(56) References cited:
- DE-A1- 2 845 647
- FR-A1- 2 872 021

## Description

### BACKGROUND

Early onset scoliosis and scoliosis in the growing spine poses a great challenge in their treatment. In progressive cases, the spine cannot usually be controlled by bracing or even casting and it will grow accentuating the deformity with all its known consequences. On the other hand, correction, fixation, and fusion of the spine will prevent further growth of the fused spine with serious effects on the development of the cardiovascular and pulmonary system, physical appearance, and psychological impacts.

Early onset scoliosis has more recently been treated surgically either by serial distractions or growth directed mechanisms. Serial distractions using "growing rod" systems have been more reliable and have achieved a more predictable outcome. These "growing rod" systems use tandem or domino connectors designed to allow periodic distractions (e.g., every few months) via surgical approach under anesthesia. Growth directed mechanisms have been used in "Luque Trolley" techniques applying segmental wires attached to the vertebrae and rods longer than the instrumented segment to allow for directed growth of the spine by forcing the spine to follow the rods. Some recent trials have used pedicle screws instead of wires - again allowing the heads of the screws attached to the vertebrae to slide along the longer rods with growth.

Both the "growing rod" and the "growth directed" mechanisms, in current systems, are far from being fully satisfactory in the treatment of early onset scoliosis. For example, the "growing rods" have to be distracted surgically every few months for many years with all the disadvantages of multiple surgeries and anesthetic administration in the pediatric age group. In addition to the problems arising from skin and soft tissue opening, the frequent force applied to distract these systems can cause implant failures in addition to the potential negative effects of forceful spinal cord distractions.

The "growth directed" and Luque Trolley type of segmental instrumentations do not require frequent distractions. These systems, however, have not been satisfactory, mainly due to their inability to control rotation, the loss of correction, and spontaneous fusion, which have led to their failure. Even after trials to replace the wires with pedicle screws, there are still many potential problems, including auto fusion after segmental exposure to insert the pedicle screws and a high possibility of jamming between the screw rod junctions preventing smooth gliding of the screws on the rod. Another problem includes the increased risk, time consumption, and radiation exposure needed to insert the large number of multilevel pedicle screws in this very young age group. Furthermore, in these systems, the amount of growth possible before another surgery is limited to the parts of the rod left protruding from the top and bottom screws.

The document FR 2 872 021 A1 discloses a device for correction of scoliosis consisting of a rod hooked at the scoliosis concavity by two fasteners and a set of means comprising two rods attached to the spine by other attachment means located at the ends of the arc defined by the curvature of the scoliosis. The rods are able to slide freely through orifices located at the ends of a part which has the shape of a U to allow guidance of said rods.

### SUMMARY

The present invention, according to some embodiments, relates to a system designed to avoid the disadvantages of the prior art and to make the best use of the power of the growth of the spine by controlling and redirecting spinal growth as well as deforming forces of the spine to allow for longitudinal growth and to correct the residual deformity. Attaching vertebral fixation points proximal and distal to the deformed area of the spine, while strongly fixing the apex of the curve, allows this system to have the maximum control of the curve, while allowing all the vertebrae included in the curve above and below the apex to grow freely. This growth is permitted and directed by one or more connectors which are inserted between these fixation points by sliding of the rods attached to the fixation points within the connectors. Apical control should be strong and reliable to counteract the main deforming forces at the apex, thereby preventing its rotation and angulation. In some embodiments, the main correction of the curve occurs at the time of insertion of the system. Then, with time and growth, the system will allow for longitudinal growth of the spine with additional correction of the curve. As the distance between the rod and the apex of the deformity is fixed, any increase in the distance between the proximal and distal fixation points of the system will lead to a proportional decrease in the scoliosis angle.

Some embodiments address a vertebral fixation system to be used in spinal deformities in the growing spine for the pediatric and adolescent age groups. In some embodiments, the system corrects the scoliosis and allows spinal growth without frequent surgeries or complex technology by directing and controlling the forces that otherwise cause the spine to deform while growing. The system is inserted, or implanted, and includes proximal, distal, and apical vertebral fixation with the use of distractible connectors between the proximal and apical vertebrae and the distal and apical vertebrae.

After insertion, the connectors, or connector assembly, of the system permit the rod, which is fixed to the vertebrae at both ends of the curve, to slide inside one or more cylindrical members to allow for spinal growth. Meanwhile, apical vertebral fixation to the system prevents the spine from rotation or angulation, thereby preventing further deformity and even inducing more correction with time. In some embodiments, the growth directed corrective process will continue until the rod(s)/connector(s) sliding limit is exhausted (e.g., after many years).

This summary is not meant to be limiting in nature. While multiple embodiments are disclosed herein, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is a schematic view of a corrective system secured along a spine tending to exhibit a defective curvature with a concave aspect, according to some embodiments.
FIG. 2 shows a first connector and portions of a first rod and a middle assembling segment of the system of FIG. 1, according to some embodiments.
FIG. 3 is a schematic view of a scoliotic spine before correction with the system of FIG. 1, according to some embodiments.
FIG. 4 shows the spine of FIG. 3 after application of the system of FIG. 1, according to some embodiments.
FIG. 5 shows the spine and system of FIG. 4 following spinal growth and elongation of the system, according to some embodiments.

As previously indicated, this description of the drawings is not meant to be limiting in nature.

### DETAILED DESCRIPTION

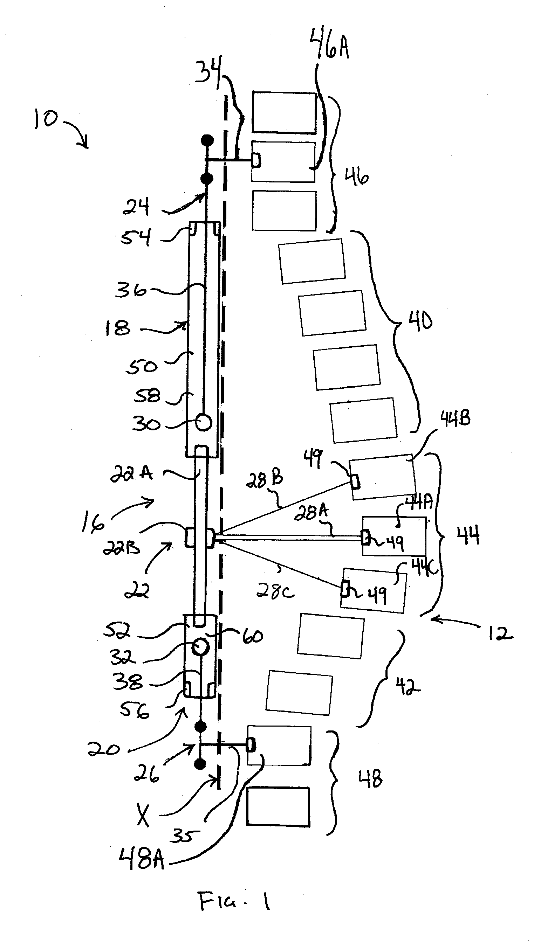

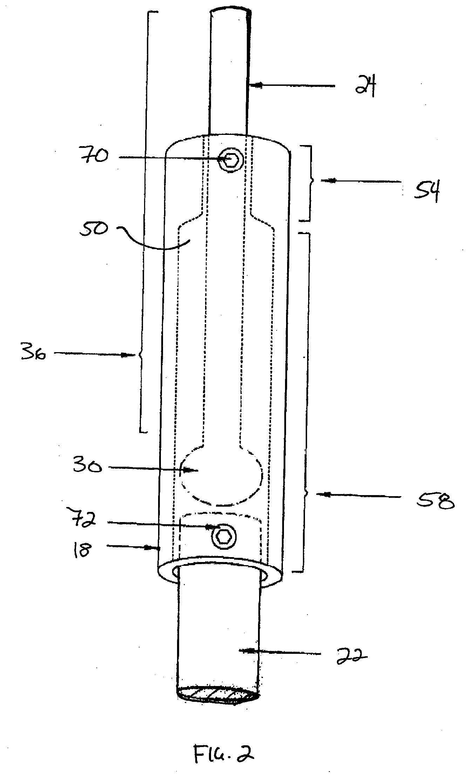

FIG. 1 is a schematic view of a system 10 for growth directed correction of a spine 12 via control of one or more apical vertebrae. The system 10 is secured to a spine 12 along a concave aspect of its defective curvature. In some embodiments, the system 10 includes a hosting connector assembly 16 including a first connector 18, a second connector 20, and a middle assembling segment 22. In the various embodiments, the system 10 further includes a first rod 24, a second rod 26, and an intermediate connector 28. FIG. 2 shows the first connector 18 and portions of the first rod 24 and the middle assembling segment 22.

The first and second rods 24, 26 are adapted to extend along the spine 12 and optionally differ in length as shown in FIG. 1, although in other embodiments the first and second rods are substantially similar in length. In some embodiments, rod length is selected to allow a desired degree of growth of the spine 12. The rods 24, 26 each optionally include an enlarged stop feature 30, 32 having a larger diameter than adjacent portions of the respective rods 24, 26. In some embodiments, the stop features 30, 32 of the rods 24, 26 are thicker, shorter portions (e.g., with smooth rounded outline) which are hosted by wider areas of the connectors 18, 20 and are allowed to slide within the respective connectors 18, 20 until they abut narrower parts of the connectors. Each of the rods 24, 26 also includes thinner longer portions 36,38.

As shown schematically in FIG. 1, the spine 12 generally includes five portions, where a defective segment of the spine 12 includes a proximal, or upper portion 40; a distal, or lower portion 42; and an apical portion, or apex 44. Above and below the defective segment 40, 42, 44, the spine 12 has a first portion 46 including one or more stabilizing vertebrae (e.g., a first vertebra 46A) and a second portion 48 including one or more stabilizing vertebrae (e.g., a second vertebra 48A). In some embodiments, the stabilizing vertebrae are substantially aligned and are optionally fused during, prior to, or after assembly of the system 10. In turn, the apical portion 44 includes one or more vertebrae at the apex of the defect (e.g., a third vertebra 44A, a fourth vertebra 44B, and a fifth vertebra 44C).

The thinner portions 36, 38 of the rods 24, 26 are adapted to host means of spinal fixation 34, 35, such as polyaxial pedicle screws, to the first and second portions 46, 48 of spine 12 at both ends of the defective segment 40, 42, 44. For example, in some embodiments, the means of spinal fixation 34, 35 include polyaxial pedicle screws used to secure the thinner longer portions 36, 38 of the rods 24, 26 to one or more vertebrae in each of the first and second portions 46, 48, respectively, of the spine 12. If desired, each of the thinner longer portions 36, 38 is secured to the first and second vertebrae 46A, 48A, respectively, of the first and second portions 46, 48. In some embodiments, one or both of the thinner longer portions 36, 38 are secured to multiple vertebrae, such as two adjacent stabilizing vertebrae of the first and second portions 46, 48, respectively (e.g., to provide additional support to the system 10).

In some embodiments, the middle assembling segment 22 includes a body portion 22A, such as a rod, a plate, or other structure for spanning between the first and second connectors 18, 20 and to which a vertebra (e.g., a third vertebra 44A in the apical portion 44) can be tensioned. The middle assembling segment 22 also optionally includes an interconnect portion 22B, such as a collar or a head of a polyaxial screw, for connecting to the body portion 22A.

In some embodiments, the intermediate connector assembly 28 includes one or more elongate members, such as first elongate member 28A, second elongate member 28B, and third elongate member 28C. The elongate members 28A, 28B, 28C optionally include one or more cables, wires, pedicle screws, rods, and/or other means for spanning between the interconnect portion 22B of the middle assembling segment 22 and the apical portion 44. The elongate members 28A, 28B, 28C are optionally connected to the third, fourth, and fifth vertebrae 44A, 44B, 44C of the apical portion 44, respectively, by fastening means 49, such as threaded fasteners, adhesives, hooks, sublaminar wires, and/or others.

The first and second connectors 18, 20 optionally differ in length as shown in FIG. 1, although in other embodiments the connectors 18, 20 are substantially similar in length. The first and second connectors 18, 20 are adapted to extend along a desired spinal segment (e.g., including the upper and lower portions 40, 42). In some embodiments, the lengths of the first and second connectors 18, 20 are selected to allow a desired amount of longitudinal growth of the spine 12, where the connectors 18, 20 are each optionally cylindrical, having inner bores 50, 52 that have narrowed, neck portions 54, 56 and wider portions 58, 60 such that the inner bores 50, 52 include two parts with different diameters.

In some embodiments, the diameters of the wider portions 58, 60 of the bores 50, 52 are larger than the diameters of the thicker, stop features 30, 32 of the rods 24, 26 to allow introduction of the rods 24, 26 into the bores 50, 52, starting with the thinner portions 36, 38 of the rods 24, 26 which are first introduced through the openings into which the body portion 22A of the middle assembling segment 22 is subsequently inserted and secured. The stop features 30, 32 of the rods 24, 26 help retain the rods 24, 26 in the inner bores 50, 52 by engaging the narrowed or necked portions 54, 56 of the connectors 18, 20 and help prevent inadvertent ejection of the rods 24, 26 from the connectors 18, 20.

In some embodiments, each of the connectors 18, 20 includes two means of fixation (e.g., set screws, pins, or others) for selectively locking a longitudinal position of the rods 24, 26 with respect to the first and second connectors 18, 20, respectively. As used herein, "selectively locking" indicates that the longitudinal position is locked and unlocked as desired using the means of fixation of the first and second connectors 18, 20. According to some embodiments, independent control of each of the upper and lower portions 40, 42 of the deformity is achieved by preselecting a desired amount that each of the first and second rods 24, 26 is allowed to travel in the respective first and second connectors 18, 20 (e.g., by selecting a length of the connectors 18, 20 and rods 24, 26) and/or by selectively locking the rods 24, 26 using the means of fixation once a desired amount of growth is achieved.

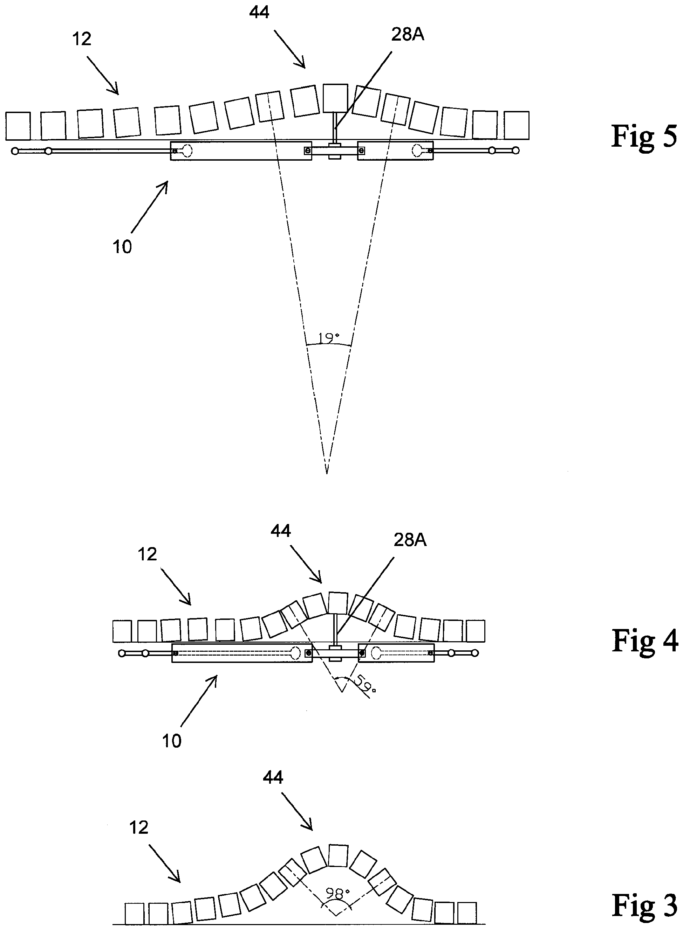

FIG. 2 shows a first means of fixation 70 and a second means of fixation 72 of the first connector 18, where according to some embodiments the second connector 20 includes similar means of fixation that operate similarly to the first and second means of fixation 70, 72 (see FIGS. 4 and 5). In the embodiment shown in FIG. 2, the first and second means of fixation 70, 72 are located at each end of the connector 18. The second means of fixation 72 (e.g., a set screw) is optionally used to fix the connector 18 to the middle assembling segment 22, the middle assembling segment 22 being received in the central bore 50 of the connector 18. The first means of fixation 70 is a temporary fixation point to fix the connector 18 to the thinner portion 36 of the rod 24 as desired. The means for fixation of the second connector 20 optionally operate similarly and, by fixing the rods 24, 26 to the connectors 18, 20, the rods 24, 26, and connectors 18, 20 can be handled as one piece for ease of use during their insertion in the index surgery. Following insertion, the first means of fixation 70 of the first connector 18 and the first means of fixation (not shown) of the second connector 20 are released (e.g., unscrewed and/or removed) at the end of the procedure to disengage the connectors 18, 20 from the rods 24, 26 to allow for gradual sliding of the rods 24, 26 within the connectors 18, 20 with growth of the spine 12.

The diameters of the narrower, or thinner portions 36, 38 of the rods 24, 26 allow the thinner portions 36, 38 of the rods 24, 26 to go through the bores 50, 52, while the thicker stop features 30, 32 prevent the rods 24, 26 from ejecting from the bores 50, 52 and limit sliding of the rods 24, 26 to a desired range. In other words, the rods 24, 26 will slide in the connectors 18, 20 with the thicker parts of the rods 24, 26 moving out into the wider parts 58, 60 of the bores 50, 52 of the connectors 18, 20 until they abut against the narrower, necked portions 54, 56 of the bores 50, 52, preventing the rods 24, 26 from further sliding. At this point, the length of the rods 24, 26 and more generally the system 10 will be exhausted and the system 10 will likely need to be adjusted by exchanging the rods 24, 26 and / or connectors 18, 20 to longer sizes.

In some embodiments, the body portion 22A of the middle assembling segment 22 is introduced into, and fixed to both wider ends of the bores 50, 52 of the connectors 18, 20. Upon assembly and fixation to the first and second vertebrae 46A, 48A, the rods 24, 26, connectors 18, 20, and middle assembling segment 22 define a correction axis X extending between the first and second vertebrae 46A, 48A. The body portion 22A of the middle assembling segment 22 is assembled to the interconnect portion 22B which hosts the intermediate connector 28. As described above, the intermediate connector 28 optionally includes elongate members 28A, 28B, 28C that include one or more of cables, wires, pedicle screws, hooks, or other means for spanning between the middle assembling segment 22 and the intermediate connector 28. The distance between the middle assembling segment 22 and the apical portion 44 can be decreased by shortening the length of this fixation tool to tension or draw the apical portion 44 (e.g., the third vertebra 44A) toward the correction axis X.

One exemplary method of assembly includes coupling the first and second rods 24, 26 with the first and second connectors 18, 20, and then coupling the first and second connectors 18, 20 together with the middle assembling segment 22. When assembled, the thinner portions 36, 38 of both rods 24, 26 extend out of the narrower openings or necked portions 54, 56 of the corresponding connectors 18, 20. The thinner portions 36, 38 may then be attached to the spine 12 proximal and distal to the spinal deformity via vertebral fixation implants (e.g., hooks, screws, or others) at the first and second vertebrae 46A, 48A. The bigger end of both rods 24, 26 (stop features 30, 32) will each be hosted inside the respective bores 50, 52 of one of the connectors 18, 20 near the wider portions 58, 60 of the bores 50, 52 and beside the middle assembling segment 22 to allow the rods 24, 26 to slide inside the bores 50, 52 during growth of the spine 12. Both wider portions 58, 60 of the bores 50, 52 of the connectors 18, 20 receive the body portion 22A of the middle assembling segment 22 which is then secured within the body portion 22A. The elongate member(s) 28A, 28B, 28C of the intermediate connector 28 are secured to the interconnect portion 22B of the middle assembling segment 22 and the elongate member(s) 28A, 28B, 28C are secured to the third, fourth, and fifth vertebrae 44A, 44B, 44C using the fastening means 49 to thereby fix and control the apical portion 44 with respect to the middle assembling segment 22.

One exemplary method of growth directed correction of the curvature with the system 10 proceeds as follows. The system 10 is applied and secured to the first portion 46 (e.g., first vertebra 46A), the second portion 48 (e.g., second vertebra 48A), and apical potion 44 (e.g., one or more of the third, fourth, and fifth vertebrae 44A, 44B, 44C), for example, after maximum correction has been achieved by surgery. Then, with growth, both bulkier ends or stop features 30, 32 of the rods 24, 26 will slide outwardly, away from the body portion 22A within the first and second connectors 18, 20 allowing for directed growth of the spine until the rods 24, 26 are exhausted and the bulkier parts, or stop features 30, 32 abut against the necked portions 54, 56 of the connectors 18, 20 and/or until the rods 24, 26 are locked at a desired position via the fixation means (e.g., set screws) of the first and second connectors 18, 20. This interaction allows for spontaneous growth (e.g., several centimeters) and many years of growth while keeping the distance between the middle assembling segment 22 and the apical portion 44. In some embodiments, the distance between the middle assembling segment 22 and the apical portion 44 is reduced using a specific instrument, such as a cable or wire tensioner (not shown).

A schematic representation of a method of growth directed correction is provided in FIGS. 3-5, where FIG. 3 shows the spine 12 having a scoliotic curve (e.g., a severe curve greater than about 90 degrees) prior to application of the system 10. FIG. 4 shows the spine 12 and the system 10 after application of the system 10. As shown in FIG. 4, and according to some embodiments, the system 10 is secured to the spine 12 with some amount of apical correction during fixation (e.g., to a curve of about 59 degrees). In some embodiments, partial correction is accomplished by drawing the apical portion 44 toward the system 10 as part of the apical fixation process. FIG. 5 shows the system 10 and spine 12 following spinal growth (e.g., a few years later) where the spine 12 and the system 10 have elongated causing growth directed correction of the spine 12 resulting gradually and spontaneously without further intervention (e.g., to a curve of about 19 degrees). In some embodiments, however, further intervention following some growth is contemplated to encourage and / or augment correction. For example, such intervention optionally includes reducing the distance between the system 10 and the apical portion 44 by tensioning and / or shortening one or more of associated elongate member(s) 28 (a single elongate member 28A is shown in FIGS. 4 and 5).

Various features and advantages of embodiments of the system 10 should be apparent from the foregoing. For example, in some embodiments, the system 10 is easy to fabricate, is low profile such that it is suitable for all ages, and efficient and effective in use. The system 10 is optionally assembled as a single construct via the temporary means of fixation between the rods 24, 26 and connectors 18, 20, promoting ease of insertion and securement to the spine. Once implanted, the system 10 is optionally designed to work over the course of multiple years without substantial intervention.

The range of indication of embodiments of the system 10 is wide enough to include any type of early onset spinal deformity of any etiology from the very young ages to the adolescent growth spurt, for example. One exemplary indication is early onset scoliosis where the system 10 is used in young children to allow for growth of the spine, trunk, chest, and lungs while preventing progression of the scoliotic curve and even correcting the curve spontaneously with growth. The system 10 can also be used in small and moderate sized curves during the adolescent period before severe progression as a kind of internal bracing to help prevent further progression of these defective curves until a child's growth spurt finishes. In some embodiments, once the growth spurt has ended, the system 10 is removed, leaving a non-fused, relatively flexible, corrected spine.

In view of the foregoing, various embodiments provide a vertebral system 10 for correction and controlled growth of the spine 12 compromising rod(s) 24, 26, a hosting connector assembly 16, and an intermediate connector 28. Embodiments include rods 24, 26 with different diameters of its both ends, where the bigger ends of the rods 24, 26 are optionally smooth to allow sliding in first and second connectors 18, 20 having end openings of different diameters. The connectors 18, 20 optionally have a wider openings to allow introduction of the rods 24, 26 starting with their thinner then thicker parts inside the connectors 18, 20. The wider opening can accommodate and be fixed to a middle assembling segment 22 of the system 10 via any stable means of fixation (e.g., set screws, threads, or others). In some embodiments, the system 10 includes a middle assembling segment 22 that includes a rod or plate which is attached to the intermediate connector 28, which is in turn secured to the apical portion 44 via vertebral fixation means (e.g., hooks, screws, wires, or other fastening means). The connectors 18, 20 provide temporary fixation (e.g., using set screws, pins, or others) to the rods 24, 26 during assembly and insertion of the system 10. The system 10 is optionally to correct spinal deformities by allowing for growth of the spine 12 and promoting further gradual correction of the deformity with growth.

In some embodiments, the system 10 is used for acute and gradual correction of spinal deformity which allows for spinal growth of the instrumented segment by elongating automatically with growth without the need for any intervention after insertion and connection to the spine 12. The system 10 includes a hosting connector assembly or assemblies 16, special rod(s) 24, 26 and intermediate connector(s) 28. The rods 24, 26 are allowed to slide inside the hosting connector assembly 16, in turn allowing for elongation of the whole system 10 and hence the instrumented part of the spine 12. A middle assembling segment 22 is fixed to the apex 44 of the deformity using an intermediate connector 28 including one or more elongate members 28A, 28B, 28C secured to the apex 44 using fastener means (e.g., pedicle screws, hooks, wires, cables, adhesives, and/or other means) to help prevent progressive rotation, angulation, or other deformity progression.

The distance between the two ends of the system 10 are able to independently increase with time and growth, while the distance between the apex 44 of the deformity and the system 10 is fixed or can be shortened by mean of continuous tension of the apical fixation (e.g., by tensioning the elongate member(s) 28A, 28B, 28C) thereby allowing for gradual spinal deformity curve correction with growth. For example, in some embodiments, first and second connector(s) 18, 20 each have a cavity made of two parts with different diameters and lengths - a longer wider part and shorter narrower one. The connector(s) 18, 20 each have one opening at each end, each opening has a different diameter which corresponds to its adjacent cavity. In some embodiments, each rod 24, 26 has a thicker (bigger diameter) shorter part at the end of the rod 24, 26 with the aim of preventing the rod 24, 26 from dislodging from the smaller end opening of the corresponding connector 18, 20 when the system 10 reaches its maximal length. Each wider cavity of the connector(s) 18, 20 can host and allow the passage of both parts of the rod(s) 24, 26 while the narrower cavity of the connector(s) 18, 20 can host only the thinner part of the rod(s) 24, 26, thereby preventing the thicker end of the rod(s) 24, 26 from passing through the corresponding end opening of the connector(s) 18, 20.

In some embodiments, the middle assembling segment 22 connects the two hosting connectors 18, 20 together by being inserted into and secured within the wider openings and cavities of the connectors 18, 20. The rod(s) 24, 26 are introduced - their thinner parts first - into the wider openings of the connectors 18, 20 and are fixed temporarily therein. The body portion 22A of the middle assembling segment 22 is then inserted into the wider ends and fixed therein to interconnect the two connectors 18, 20 together. In some embodiments, the body portion 22A of the middle assembling segment 22 is a rod shaped, or contoured to conform with a desired shape of the spine 12 in order to promote a proper sagittal contour of the spine 12 and decrease an incidence of implant failure, for example. The middle assembling segment 22 is secured to the apical portion 44 by the intermediate connector 28, which includes fastening means such as pedicle screws, hooks, wires, cables, and/or other fastening means for fastening to the vertebrae at the apex 44 of the deformity. The connector(s) 18, 20 have means of fixation (e.g., set screw, pins, and/or others) proximate each end - at the wider end to fix the connectors 18, 20 to the middle assembling segment 22 and at the narrower end to fix the thinner part of the rods 24, 26 temporarily during assembly and insertion and attachment of the system 10 to the spine 12. In some embodiments, the temporary means of fixation, or selective locking means, are removed at the end of the procedure to allow one or both of the rods 24, 26 to slide in the connectors 18, 20 and to allow the system 10 to elongate.

As referenced above, the system 10 optionally facilitates independent, separate control of each of the upper and lower portions 40, 42 of a deformity, those upper and lower portions 40, 42 being situated proximal and distal to an apical portion 44 of the deformity. For example, a distance between each end of the system 10 and the apical portion 44 increases independently with time and growth of the spine 12, while the distance between the apical portion 44 and the system 10 is generally fixed or selectively adjusted (e.g., by tensioning the apical portion 44 toward the hosting connector assembly 16) allowing for gradual or gross spinal deformity curve correction. The first and second connectors 18, 20 optionally have different lengths, (e.g., to facilitate differing, independent, and preplanned control of the permissible growth and correction of the upper and lower portions 40, 42 of the spine 12). In some methods of differing, independent, and preplanned control, a deformity angle and number of vertebrae included in each of the upper and lower portions 40, 42 are taken into consideration in determining an appropriate amount of travel between the first rod 24 and the first connector 18 and between the second rod 26 and the second connector 20, where each of the first and second rods 24, 26 is able to slide independently of the other rod inside its corresponding connector to facilitate independent elongation of the system 10 along the instrumented portions of the spine 12 above and below the apical portion 44. In some methods of correction, the second mean of fixation of each of the first and second connectors 18, 20 can, at any time after the application of the system 10, can be tightened to limit further elongation of the corresponding upper or lower portion 40, 42 of the spine 12. By including means for selectively limiting growth of the upper or lower portions 40, 42 of the spine 12, the system 10 is further adapted to promote independent correction of each of the upper and lower portions 40, 42 as desired.

## Claims

1. A system (10) for growth directed correction of a spine (12) via apical vertebral control, the system (10) comprising:
a first rod (24) extending from a first end to a second end and being adapted to be secured to a first vertebra proximate the first end of the first rod (24);
a second rod (26) extending from a first end to a second end and being adapted to be secured to a second vertebra proximate the first end of the second rod (26);
a hosting connector assembly (16) adapted to combine with the first rod (24) and the second rod (26) to define a correction axis extending adjacent the spine (12), **characterized in that** the hosting connector assembly (16) including:
a first connector (18) including an inner bore (50) receiving the second end of the first rod (24) and slidably connected to the second end of the first rod (24), the first connector (18) including a first locking member for selectively locking an axial position of the first rod (24) relative to the first connector (18);
a second connector (20) connected to the second end of the second rod (26); and
an intermediate connector (28) secured to the hosting connector assembly (16) and extending away from the longitudinal axis of the system (10), the intermediate connector (28) being adapted to be secured to an intermediate vertebra located between the first and second vertebrae to selectively lock a distance between the intermediate vertebra and the correction axis.

2. The system (10) of claim 1, wherein the second connector (20) is slidably connected to the second end of the second rod (26), the second connector (20) comprising a second locking member for selectively locking an axial position of the second rod (26) relative to the second connector (20).

3. The system (10) of claim 1, wherein the first end of the first rod (24) and the first end of the second rod (26) are expandable away from one another.

4. The system (10) of claim 1, wherein the first connector (18) is substantially cylindrical and defines a central bore (50), the central bore (50) having a narrowed portion (54).

5. The system (10) of claim 4, wherein proximate the second end of the first rod (24), the first rod (24) defines an enlarged stop feature (30) for engaging the narrowed portion (54) of the bore (50) for retaining the first rod (24) in the bore (50).

6. The system (10) of claim 1, wherein the intermediate connector (28) is secured relative to the correction axis and is adapted to tension the intermediate vertebra to the correction axis during spinal growth.

7. The system (10) of claim 1, wherein the intermediate connector (28) includes at least one of a wire, a hook, a pedicle screw, and a cable.

8. The system (10) of claim 1, wherein the intermediate connector (28) is centrally located between the first and second rods (24, 26) and such that the intermediate connector (28) is adapted to be secured to an apical vertebra of a defective curvature.

9. The system (10) of claim 1, wherein the first and second connectors (18, 20) each include substantially cylindrical bodies, the substantially cylindrical bodies each defining central bores (50, 52) for slidably receiving the first and second rods (24, 26), respectively.

10. The system (10) of claim 9, wherein the first and second connectors (18, 20) are connected by a middle assembling segment (22) extending between the first and second connectors (18, 20), the middle assembling segment (22) being secured in the central bores (50, 52) of the first and second connectors (18, 20), respectively.

## Patentansprüche

1. System (10) zur wachstumsausgerichteten Korrektur einer Wirbelsäule (12) über eine apikale vertebrale Steuerung, wobei das System (10) aufweist:
einen ersten Stab (24), der sich von einem ersten Ende zu einem zweiten Ende erstreckt und eingerichtet ist, an einem ersten Wirbel nahe dem ersten Ende des ersten Stabs (24) befestigt zu werden;
einen zweiten Stab (26), der sich von einem ersten Ende zu einem zweiten Ende erstreckt und eingerichtet ist, an einem zweiten Wirbel nahe dem ersten Ende des zweiten Stabs (26) befestigt zu werden;
eine aufnehmende Verbindungsanordnung (16), die eingerichtet ist, sich mit dem ersten Stab (24) und dem zweiten Stab (26) zu verbinden, um eine Korrekturachse zu definieren, die sich benachbart zur Wirbelsäule (12) erstreckt,
**dadurch gekennzeichnet, dass** die aufnehmende Verbindungsanordnung (16) aufweist:
einen ersten Verbinder (18), der eine Innenbohrung (50) aufweist, die das zweite Ende des ersten Stabs (24) aufnimmt und verschiebbar mit dem zweiten Ende des ersten Stabs (24) verbunden ist, wobei der erste Verbinder (18) ein erstes Verriegelungselement zum selektiven Verriegeln einer axialen Position des ersten Stabs (24) relativ zum ersten Verbinder (18) aufweist;
einen zweiten Verbinder (20), der mit dem zweiten Ende des zweiten Stabs (26) verbunden ist; und
einen Zwischenverbinder (28), der an der aufnehmenden Verbindungsanordnung (16) befestigt ist und sich von der Längsachse des Systems (10) weg erstreckt, wobei der Zwischenverbinder (28) eingerichtet ist, an einem Zwischenwirbel befestigt zu werden, der sich zwischen dem ersten und zweiten Wirbel befindet, um selektiv einen Abstand zwischen dem Zwischenwirbel und der Korrekturachse zu verriegeln.

2. System (10) nach Anspruch 1, wobei der zweite Verbinder (20) verschiebbar mit dem zweite Ende des zweiten Stabs (26) verbunden ist, wobei der zweite Verbinder (20) ein zweites Verriegelungselement zum selektiven Verriegeln einer axialen Position des zweiten Stabs (26) relativ zum zweiten Verbinder (20) aufweist.

3. System (10) nach Anspruch 1, wobei das erste Ende des ersten Stabs (24) und das erste Ende des zweiten Stabs (26) voneinander weg ausdehnbar sind.

4. System (10) nach Anspruch 1, wobei der erste Verbinder (18) im Wesentlichen zylindrisch ist und eine Mittelbohrung (50) definiert, wobei die Mittelbohrung (50) einen verengten Abschnitt (54) aufweist.

5. System (10) nach Anspruch 4, wobei nahe dem zweiten Ende des ersten Stabs (24) der erste Stab (24) ein aufgeweitetes Stoppmerkmal (30) zum Eingriff mit dem verengten Abschnitt (54) der Bohrung (50) zum Halten des ersten Stabs (24) in der Bohrung (50) definiert.

6. System (10) nach Anspruch 1, wobei der Zwischenverbinder (28) relativ zur Korrekturachse befestigt und eingerichtet ist, den Zwischenwirbel während des Wirbelsäulenwachstums zur Korrekturachse zu ziehen.

7. System (10) nach Anspruch 1, wobei der Zwischenverbinder (28) einen Draht und/oder einen Haken und/oder eine Pedikelschraube und/oder ein Drahtseil aufweist.

8. System (10) nach Anspruch 1, wobei der Zwischenverbinder (28) in der Mitte zwischen dem ersten und zweiten Stab (24, 26) und so angeordnet ist, dass der Zwischenverbinder (28) eingerichtet ist, an einem apikalen Wirbel einer fehlerhaften Krümmung befestigt zu werden.

9. System (10) nach Anspruch 1, wobei der erste und zweite Verbinder (18, 20) jeweils im Wesentlichen zylindrische Körper aufweisen, wobei die im Wesentlichen zylindrischen Körper jeweils Mittelbohrungen (50, 52) zum verschiebbaren Aufnehmen des ersten bzw. zweiten Stabs (24, 26) definieren.

10. System (10) nach Anspruch 9, wobei der erste und zweite Verbinder (18, 20) durch ein mittleres Montagesegment (22) verbunden sind, das sich zwischen dem ersten und zweiten Verbinder (18, 20) erstreckt, wobei das mittlere Montagesegment (22) jeweils in den Mittelbohrungen (50, 52) des ersten und zweiten Verbinders (18, 20) befestigt ist.

## Revendications

1. Système (10) pour la correction d'une colonne vertébrale (12) à des fins de croissance au moyen d'un contrôle apical vertébral, ledit système (10) comprenant :
une première tige (24) s'étendant d'une première extrémité à une deuxième extrémité et prévue pour être fixée à une première vertèbre à proximité de la première extrémité de la première tige (24) ;
une deuxième tige (26) s'étendant d'une première extrémité à une deuxième extrémité et prévue pour être fixée à une deuxième vertèbre à proximité de la première extrémité de la deuxième tige (26) ;
un ensemble de connecteurs hôte (16) prévu pour être associé à la première tige (24) et à la deuxième tige (26) pour définir un axe de correction s'étendant de manière adjacente à la colonne vertébrale (12), **caractérisé en ce que** l'ensemble de connecteurs hôte (16) comprend :
un premier connecteur (18) présentant un alésage intérieur (50) recevant la deuxième extrémité de la première tige (24) et raccordé de manière coulissante à la deuxième extrémité de la première tige (24), ledit premier connecteur (18) comportant un premier élément de fixation pour la fixation sélective d'une position axiale de la première tige (24) par rapport au premier connecteur (18) ;
un deuxième connecteur (20) raccordé à la deuxième extrémité de la deuxième tige (26) ; et
un connecteur intermédiaire (28) fixé à l'ensemble de connecteurs hôte (16) et s'étendant à distance de l'axe longitudinal du système (10), ledit connecteur intermédiaire (28) étant prévu pour être fixé à une vertèbre intermédiaire située entre la première et la deuxième vertèbres pour maintenir sélectivement une distance entre la vertèbre intermédiaire et l'axe de correction.

2. Système (10) selon la revendication 1, où le deuxième connecteur (20) est raccordé de manière coulissante à la deuxième extrémité de la deuxième tige (26), ledit deuxième connecteur (20) comprenant un deuxième élément de fixation pour la fixation sélective d'une position axiale de la deuxième tige (26) par rapport au deuxième connecteur (20).

3. Système (10) selon la revendication 1, où la première extrémité de la première tige (24) et la première extrémité de la deuxième tige (26) peuvent être éloignées l'une de l'autre.

4. Système (10) selon la revendication 1, où le premier connecteur (18) est sensiblement cylindrique et définit un alésage central (50), ledit alésage central (50) présentant une partie resserrée (54).

5. Système (10) selon la revendication 4, où à proximité de la deuxième extrémité de la première tige (24), la première tige (24) définit un élément de butée large (30) destiné à buter contre la partie resserrée (54) de l'alésage (50) pour maintenir la première tige (24) dans l'alésage (50).

6. Système (10) selon la revendication 1, où le connecteur intermédiaire (28) est fixé par rapport à l'axe de correction et est prévu pour mettre sous tension la vertèbre intermédiaire par rapport à l'axe de correction pendant la croissance vertébrale.

7. Système (10) selon la revendication 1, où le connecteur intermédiaire (28) comprend un fil métallique et/ou un crochet et/ou une vis pédiculaire et/ou un câble.

8. Système (10) selon la revendication 1, où le connecteur intermédiaire (28) est situé centralement entre la première et la deuxième tiges (24, 26) de manière à permettre la fixation dudit connecteur intermédiaire (28) à une vertèbre apicale à courbe défectueuse.

9. Système (10) selon la revendication 1, où le premier et le deuxième connecteurs (18, 20) ont chacun un corps sensiblement cylindrique définissant des alésages centraux (50, 52) respectifs pour la réception coulissante de la première et de la deuxième tiges (24, 26).

10. Système (10) selon la revendication 9, où le premier et le deuxième connecteurs (18, 20) sont raccordés par un segment d'assemblage médian (22) s'étendant entre le premier et le deuxième connecteurs (18, 20), ledit segment d'assemblage médian (22) étant fixé dans les alésages centraux (50, 52) respectifs du premier et du deuxième connecteurs (18, 20).
